# EUROPEAN PATENT APPLICATION

(11) **EP 3 421 589 A1**
(43) Date of publication of application: **02.01.2019**
(21) Application number: 17756806.0
(22) Date of filing: 22.02.2017
(51) Int. Cl.: C12N 5/0775, C12N 5/0789, A61K 35/28

(54) **COMPOSITION AND METHOD FOR IMPROVING EFFICACY OF STEM CELLS**

(30) Priority: 23.02.2016 KR 20160021374
(71) Applicant: University - Industry Cooperation Group of Kyung Hee University, Yongin-si, Gyeonggi-do 17104 (KR)
(72) Inventor: PARK, Ki-Sook, Seoul 06374 (KR); YU, Jinyeong, Seoul 02446 (KR)
(74) Representative: Icosa
(86) International application number: PCT/KR2017/001960
(87) International publication number: WO 2017/146468

(57) **Abstract**

The present invention relates to a method for increasing one or more selected from the group consisting of mobility, an engraftment ability, a CFU-F forming ability, an anti-inflammatory immunosuppression induction function, and an angiogenesis-promoting ability of stem cells, comprising treating stem cells with a short-chain fatty acid or a salt thereof. According to the present invention, the method can readily prepare, in large quantities, stem cells having high mobility, an excellent colony formation ability, and an excellent engraftment ability so as to have excellent biocompatibility to human body, using a low-cost short-chain fatty acid derived from human body or a salt thereof.

## Description

### [Technical Field]

The present invention relates to a composition for enhancing characteristics of stem cells, such as mobility, an engraftment ability, an ability of forming colony-forming unit fibroblasts (CFU-F), an anti-inflammatory immunosuppression inducing function, and an angiogenesis-promoting ability of stem cells, and a method thereof.

### [Background Art]

Mesenchymal stem cells (hereinafter, MSCs) are stem cells present in bone marrow, *etc.* along with hematopoietic stem cells. MSCs have the potential to differentiate into various cell types (*e.g.,* bone cells, chondrocytes, adipocytes, myoblasts, hepatocytes, myocytes, neurons, *etc*.) when suitable culture conditions for proliferation and expansion are provided during *in vitro* culture. For this reason, studies of MSCs for tissue regeneration have been performed in many fields such as cell biology and tissue engineering, and some clinical trial studies are also underway.

As described above, stem cells have a high potential for cell therapy in that they have the multipotency and are able to induce differentiation into the desired cells in damaged cells. However, the survival rate of transplanted cells is not high at the current development level, and thus it is difficult to find successful examples with a broad spectrum and stability in real clinical application.

For the above reasons, it is necessary to provide appropriate stimulation to stem cells to activate them into a state suitable for transplantation or change their characteristics.

For example, SCs are cultured in the *in vitro* state establishing characteristics similar to those of fibroblasts while forming colonies, and thus, these stem cells were named as colony-forming unit fibroblasts (hereinafter, CFU-Fs). It has been identified that CFU-Fs are formed such that a single mesenchymal stem cell is proliferated into a colony. That is, CFU-Fs means the number of MSCs and represents the high effect of MSCs. Therefore, a higher number of CFU-Fs means that a greater amount of cells with excellent MSCs characteristics can be obtained, and thus, it should be possible that CFU-Fs can be produced on a large scale when cultured in the *in vitro* state.

Additionally, it is necessary to promote the mechanisms of migration and induction of stem cells so that stem cells can rapidly act in the lesion after transplantation, or promote rapid migration of stem cells or improve their engraftment ability *in vivo.*

Meanwhile, it is known that once MSCs are activated, various immunosuppressive molecules and growth factors that promote tissue regeneration and maintenance of immune homeostasis are expressed thereby inducing an anti-inflammatory immunosuppression effect. MSCs can induce an immunoregulatory effect (*e.g.,* an immunosuppressive effect, *etc*.) by secreting various kinds of cytokines (*e.g.,* IL-10, IL6, TGFβ, chemokines, CL-2/MCP-1, CCL5/RANTES, IDO, VEGF, ICAM, PGE2, *etc.*) in immune responses. Additionally, inflammatory cytokines due to inflammation during an inflammatory reaction can recruit MSCs in the inflammatory region, and in particular, MSCs may be transdifferentiated in the form of a tissue-specific cell or exhibit an effect of local recovery through secretion of factors having an anti-inflammatory function. Such characteristics of MSCs in immune responses imply potential clinical applicability of MSCs (*see* Kyurkchiev D et al., World Journal of Stem Cells, 2014 Nov., 6(5); 552 to 570; S Ma et al., Cell Death and Differentiation (2014) 21, 216 to 225; and Madrigal et al. Journal of Translational Medicine 2014, 12: 260).

Angiogenesis is a process of forming new capillary blood vessels where endothelial cells in the existing blood vessels decompose extracellular matrices (ECMs), migrate, divide, and differentiate. Angiogenesis is involved in various physiological and pathological phenomena such as wound healing, embryogenesis, tumor formation, chronic inflammation, obesity, *etc.*

Angiogenesis may be considered as a phenomenon essential for wound healing or tissue regeneration. For example, necrosis, ulceration, and ischemia due to failure of blood vessel formation may cause malfunction of the tissue or organ or may become a cause of death. Diseases such as arteriosclerosis, myocardial infarction, and angina are also caused by poor blood supply.

It is known that activated MSCs are involved in wound healing such that the activated MSCs express growth factors including VEGF and stimulate angiogenesis and differentiation of vascular endothelial cells thereby promoting MSCs-mediated tissue regeneration (*see* Madrigal et al. Journal of Translational Medicine 2014, 12:260).

However, little is known about the methods for the enhancement of an engraftment ability, a CFU-F forming ability, proliferation and mobility, an anti-inflammatory function, an immunosuppressive function, and angiogenesis of these stem cells *ex vivo* and *in vitro* as well as *in vivo.*

Accordingly, there is a need for the identification of novel factors capable of enhancing the effects of stem cells for the use and enhancement of therapy efficiency of stem cells, and a need for the development of relevant technologies.

### [Prior Art]

### [Patent Document]

KR Patent Application Publication No. 10-2016-0009420 (published January 26, 2006)
KR Patent Application Publication No. 10-2011-0044722 (published April 29, 2011)

### [Disclosure]

### [Technical Problem]

The present invention provides a method for enhancing one or more stem cell functions selected from the group consisting of mobility, an engraftment ability, a CFU-F forming ability, an anti-inflammatory immunosuppression inducing function, and an angiogenesis-promoting ability of stem cells, which includes treating stem cells with a short-chain fatty acid or a salt thereof.

The present invention also provides a composition for enhancing one or more stem cell functions selected from the group consisting of mobility, an engraftment ability, a CFU-F formation ability, an anti-inflammatory immunosuppression inducing function, and an angiogenesis-promoting ability of stem cells, containing a short-chain fatty acid or a salt thereof as an active ingredient.

The present invention also provides stem cells prepared by the above method.

The present invention also provides a cell therapy composition containing the stem cells prepared by the above method.

The present invention also provides a method for promoting migration of bone marrow-derived hematopoietic stem cells into the peripheral blood in a subject excluding humans, which includes administering a short-chain fatty acid or a salt thereof to the subject.

The present invention also provides a method for enhancing mobility of stem cells which includes treating a short-chain fatty acid or a salt thereof to stem cells.

The present invention also provides a composition for enhancing mobility of stem cells which contain a short-chain fatty acid or a salt thereof as an active ingredient.

### [Technical Solution]

The present inventors have made efforts to maximize the characteristics of stem cells suitable for transplantation by activating them through provision of appropriate stimulation thereto or by changing their characteristics. As a result, they have discovered that the treatment of stem cells with a short-chain fatty acid or salts thereof can significantly enhance the mobility, colony-forming ability, engraftment ability, anti-inflammatory immunosuppression inducing function, and an angiogenesis-promoting ability of stem cells thereby completing the present invention.

The present invention provides a method for enhancing one or more stem cell functions selected from the group consisting of mobility, an engraftment ability, a CFU-F forming ability, an anti-inflammatory immunosuppression inducing function, and an angiogenesis-promoting ability of stem cells, which includes treating stem cells with a short-chain fatty acid or a salt thereof.

According to the above method of enhancement of the present invention, the pretreatment of stem cells *in vitro* and *in vivo* can significantly enhance mobility, an engraftment ability by transplantation of stem cells, a CFU-F forming ability, an anti-inflammatory immunosuppression inducing function, and an angiogenesis-promoting ability of stem cells. Therefore, the present invention provides stem cells as a cell therapy product having characteristics suitable for transplantation by activating stem cells to be in a state suitable for transplantation and appropriately changing the characteristics of these stem cells to be suitable for the human body.

In the present invention, the short-chain fatty acid or a salt thereof is one or more selected from acetic acid, propionic acid, butyric acid, caproic acid (C₆), caprylic acid (C₈), capric acid (C₁₀), lauric acid (C₁₂), myristic acid (C₁₄), palmitic acid (C₁₆), stearic acid (C₁₈), oleic acid (C_{18:1}) elaidic acid (C_{18:1}), linoleic acid (C_{18:2}), α-linolenic acid (C_{18:3}), γ-linolenic acid (C_{18:3}), arachidonic acid (C_{20:3}), eicosapentaenoic acid (C_{20:5}), docosahexaenoic acid (C_{22:6}), 5,6-epoxyeicosatrienoic acid, and 8,9-epoxyeicosatrienoic acid, and salts thereof; preferably fatty acid having up to 6 carbon atoms or a salt thereof; and more preferably propionic acid, butyric acid, and a salt thereof, which are human-derived and are thus safe for use in humans.

The salt means salts commonly used in the pharmaceutical industry. For example, the salt is an inorganic ionic salt prepared using calcium, potassium, sodium, and magnesium, and preferably, and preferably a sodium salt.

The stem cells usable in the present invention include without limitation embryonic stem cells, adult stem cells, induced pluripotent stem cells, embryonic germ cells, and embryonic tumor cells, preferably multipotent adult stem cells, more preferably hematopoietic stem cells and mesenchymal stem cells (MSCs), and most preferably bone marrow-derived MSCs and hematopoietic stem cells.

As used herein, the term "mobility" refers to the enhancement of migration activity of stem cells, and it includes all of the effects, such as an effect of increasing the ability of stem cells to migrate from a graft site to a damaged site, an effect of increasing the ability of stem cells to migrate from bone marrow to peripheral blood, an effect of increasing the ability of stem cells to migrate from peripheral blood to a certain tissue or organ (*e.g.,* lymph nodes, heart, lungs, livers, skin, spleens, small and large intestines, stomach, pancreas, *etc*.), *etc.* For example, the accelerated migration from a graft site to a damaged site enables achievement of effects, such as reducing the number of effective stem cells for transplantation, a subsequent decrease in the *ex vivo* culture period of stem cells, an increase of safety of stem cells, and reduction of manufacturing cost of stem cells. Additionally, the acceleration of the migration of stem cells from bone marrow to peripheral blood enables easier separation of a large number of the migrated stem cells and thus it becomes easy to collect stem cells for the transplantation of bone marrow-derived stem cell. Additionally, the mobility of stem cells has an advantage in that the migration from peripheral blood to peripheral organs can be accelerated thereby increasing the therapeutic effect.

As used herein, the term "engraftment ability" refers to the ability of the transplanted stem cells to be able to replace the subsequent cells into which the stems cells were differentiated or the cells produced *in vivo* after transplantation; or the ability of the injected cells to be able to replace lost or damaged cells. According to the enhancement of the engraftment ability, advantages, such as reduction in the number of effective stem cells for transplantation, a subsequent decrease in the *ex vivo* culture period of stem cells, an increase of safety of stem cells, and reduction of manufacturing cost of stem cells, can be obtained.

As used herein, the term "CFU-F forming ability" means that a single mesenchymal stem cell forms a colony as a result of proliferation. According to the enhancement of the CFU-F forming ability, advantages, such as safe and effective expansion *ex vivo* and *in vitro* due to the enhancement of extension of sternness and proliferation ability of stem cells can be obtained.

As used herein, the term "anti-inflammatory immunosuppression inducing function", which is the provision of an inhibitory signal that helps control or suppress inflammatory or an immune response, refers to a function that induces blocking all or part of the inflammatory response or immune stimulation; deteriorating, preventing, or delaying immunological activity; or reduction of antigen presentation, T cell activation, T cell differentiation, expression of T cell function, secretion or production of cytokines, lysis of target cells, *etc.*

According to the enhancement of the anti-inflammatory immunosuppression inducing function, there is an advantage in that it is possible to culture a stem cell source of a stem cell therapy product with further enhancement on effectiveness for various autoimmune diseases or inflammatory diseases. In addition, as it is possible to culture stem cells with high expression of materials capable of inducing anti-inflammatory immunosuppression, advantages such as reducing the number of effective stem cells for transplantation, a subsequent decrease in the *ex vivo* culture period of stem cells, an increase of safety of stem cells, and reduction of manufacturing cost of stem cells, can be obtained.

The autoimmune diseases or inflammatory diseases may be selected from the group consisting of rheumatoid arthritis, asthma, dermititis, psoriasis, cystic fibrosis, multiple sclerosis, systemic lupus erythematosus, Sjogren syndrome, Hashimoto thyroiditis, polymyositis, scleroderma, Addison disease, vitiligo, pernicious anemia, glomerulonephritis, pulmonary fibrosis, inflammatory bowel dieseses, ulcerative colitis, autoimmune diabetes, diabetic retinopathy, rhinitis, ischemia-reperfusion injury, post-angioplasty restenosis, chronic obstructive pulmonary diseases (COPD), Graves disease, gastrointestinal allergies, conjunctivitis, atherosclerosis, coronary artery disease, angina, and small artery disease.

As used herein, the term "angiogenesis-promoting ability" refers to the process where new blood vessels are formed, that is, a function of promoting the development and differentiation of new blood vessels into cells, tissues or organs. In the present invention, the promotion of angiogenesis includes the promotion of vascular regeneration, vascular restoration, and vascular differentiation associated with the angiogenic process, such as activation of endothelial cells, migration, proliferation, reformation of matrix, and cell stabilization.

By the angiogenesis-promoting ability, it is possible to promote vascular regeneration, vascular restoration, and vascular differentiation in tissues with reperfusion injury (*e.g.,* damaged myocardium in ischemic myocardial infarction, brain injury due to stroke, *etc.*).

Further, according to the present invention, stem cells with an enhanced ability of tissue regeneration due to the angiogenesis-promoting ability can be cultured. In addition, as it is possible to culture stem cells with high expression of materials capable of inducing the promotion of angiogenesis, advantages such as reducing the number of effective stem cells for transplantation, a subsequent decrease in the *ex vivo* culture period of stem cells, an increase of safety of stem cells, and reduction of manufacturing cost of stem cells, can be obtained.

As described above, the stem cells with enhanced abilities in the mobility, engraftment ability, CFU-F forming ability, anti-inflammatory immunosuppression inducing function, and angiogenesis-promoting ability can have excellent therapeutic effects as a cell therapy product.

The method according to the present invention may be performed *ex vivo* or *in vitro.*

In the present invention, the step of treating a short-chain fatty acid or a salt thereof to stem cells means that the stem cells cultured in medium are treated with the short-chain fatty acid or a salt thereof.

In the present invention, the medium refers to that where growth and survival *in vitro* can be supported, and all of the conventional media suitable for cell culture used in the art can be included. The medium and culture conditions can be selected according to the type of cells. The medium used in culture is preferably a cell culture minimum medium (CCMM, which generally contains carbon sources, nitrogen sources, and trace elements. Such a cell culture minimum medium may include, for example, Dulbecco's modified Eagle's medium (DMEM), minimal essential medium (MEM), basal medium Eagle (BME), RPMI1640, F-10, F-12, α minimal essential medium (αMEM), Glasgow's minimal essential medium (GMEM), Iscove's modified Dulbecco's medium (IMDM), but the medium is not limited thereto. Additionally, the medium may contain antibiotics such as penicillin, streptomycin, gentamicin, *etc.*

According to an embodiment of the present invention, the medium is DMEM or RPMI1640, and the medium further contains serum component(s) and an antibiotic(s).

In the present invention, the short-chain fatty acid or a salt thereof must be contained in the above medium at an effective concentration.

In the present invention, the effective concentration refers to a sufficient amount of a short-chain fatty acid or a salt thereof at which stem cells can exhibit the enhancement of mobility, an engraftment ability, a CFU-F forming ability, an anti-inflammatory immunosuppression inducing function, and an angiogenesis-promoting ability. Since stem cells do not exhibit activity at a concentration below the effective concentration but at a concentration above the effective concentration, the short-chain fatty acid or a salt thereof must be used within the effective concentration. In the present invention, the concentration of the short-chain fatty acid or a salt thereof for treatment is preferably in the range of 0.1 mM to 100 mM, and more preferably 1 mM to 20 mM.

Additionally, in the present invention, the short-chain fatty acid or a salt thereof may be treated for 3 hours to 14 days, and more preferably 12 to 48 hours.

The step of treating stem cells with a short-chain fatty acid or a salt thereof may be performed in normal serum medium or may be performed after transferring the stem cells subcultured in serum medium into serum-free medium. The cultivation in serum-free medium may be performed after removing the culture of stem cells cultured in serum medium followed by washing the cells with phosphate buffer.

The present invention preferably provides a method for enhancing mobility of stem cells which includes treating stem cells with a short-chain fatty acid or a salt thereof.

According to the method for enhancing mobility of stem cells of the present invention, the treatment of stem cells with a short-chain fatty acid or a salt thereof can significantly enhance mobility of the stem cells *in vitro* and *in vivo.* Therefore, the present invention provides stem cells with characteristics suitable for a cell therapy product can be provided by activating the stem cells to be suitable for transplantation followed by modifying the characteristics of the stem cells to be suitable for the human body.

The present invention also provides a composition for enhancing one or more stem cell functions selected from the group consisting of mobility, an engraftment ability, a CFU-F forming ability, an anti-inflammatory immunosuppression inducing function, and an angiogenesis promoting ability of stem cells, containing a short-chain fatty acid or a salt thereof as an active ingredient. The details with regard to the composition, containing a short-chain fatty acid or a salt thereof as an active ingredient, for enhancing stem cell functions of mobility, an engraftment ability, CFU-F forming ability, anti-inflammatory immunosuppression inducing function, and angiogenesis promoting ability of stem cells, include all of the details related to the method for enhancing one or more stem cell functions selected from the group consisting of mobility, an engraftment ability, CFU-F forming ability, anti-inflammatory immunosuppression inducing function, and angiogenesis promoting ability of stem cells.

The treatment of stem cells with the composition for enhancing mobility, an engraftment ability, and a CFU-F forming ability enables an increase of the mobility of stem cells, an excellent colony-forming ability, and an easy, large-scale, and low-cost production of stem cells suitable for transplantation to the human body.

Preferably, the present invention provides a composition for enhancing mobility of stem cells containing a short-chain fatty acid or a salt thereof as an active ingredient.

Additionally, the present invention provides stem cells prepared by a method for enhancing one or more stem cell functions selected from the group consisting of mobility, an engraftment ability, a CFU-F forming ability, an anti-inflammatory immunosuppression inducing function, and an angiogenesis promoting ability of stem cells, which includes treating stem cells with a short-chain fatty acid or a salt thereof. Preferably, the present invention provides stem cells prepared by the method for enhancing mobility of stem cells.

The stem cells treated with a short-chain fatty acid or a salt thereof have such characteristics that their mobility, an engraftment ability, and CFU-F forming ability are increased, and their anti-inflammatory immunosuppression inducing function and angiogenesis-promoting ability are exhibited; and action effects (*e.g.,* increases of mobility of stem cells from a transplanted area to a lesion, mobility of stem cells from the bone marrow to the peripheral blood, mobility of stem cells from the peripheral blood to a certain tissue or organ (*e.g.,* lymph nodes, heart, lungs, livers, skin, spleens, small and large intestines, stomach, pancreas, *etc.*), engraftment ability, *etc*.) are provided, thus being useful for use as a cell therapy product.

The present invention further provides a cell therapy composition containing the stem cells prepared by the above method.

The stem cells of the present invention themselves, which have enhanced functions of mobility, an engraftment ability, a CFU-F forming ability, an anti-inflammatory immunosuppression inducing function, and an angiogenesis-promoting ability, can be used for treating diseases. These stem cells may be appropriately used for treating diseases by injection, infusion, or transplantation into an animal body *in vivo* followed by subsequent rapid migration to a lesion, promoted to migrate through the peripheral blood, or by direct contact with a certain type of cell population followed by subsequent differentiation into a certain type of cells. The types of diseases that can be treated by these stem cells are not limited.

Preferably, the cells with an enhanced stem cell ability are in the form of a cell composition containing one or more diluents, which can protect and maintain cells and facilitate easier use at the time of injection, infusion, or transplantation to desired tissue. The diluent may contain buffer (*e.g.,* physiological saline, phosphate buffered saline (PBS), Hank's balanced salt solution (HBSS), *etc*.), serum, blood components, *etc.*

The administration dose may be in an amount of 10⁵ cells/kg to 10⁹ cells/kg of body weight, preferably 5 × 10⁵ cells/kg to 10⁸ cells/kg of body weight, and may be administered once or in several divided doses. However, it should be understood that the actual dose of the active ingredient must be determined in light of various relevant factors (*e*.g*.,* disease to be treated, severity of disease, administration route, patient's body weight, age, and sex, *etc*.)*.* Therefore, the administration dose does not limit the scope of the present invention in any manner.

The present invention further provides a method for promoting migration of bone marrow-derived hematopoietic stem cells into the peripheral blood in a subject excluding humans, which includes administering a short-chain fatty acid or a salt thereof to the subject.

According to the method for promoting migration of bone marrow-derived hematopoietic stem cells into the peripheral blood, the method has an advantage in that a large number of the migrated stem cells can easily be isolated from the peripheral blood by promoting the migration of hematopoietic stem cells from the bone marrow to the peripheral blood, and thus, it is easy to collect stem cells for transplantation of bone marrow-derived stem cells, and the effect of treating diseases can be increased by such a promotion of migration. In particular, according to the method for promoting migration of bone marrow-derived hematopoietic stem cells into the peripheral blood, the method has an advantage in that autologous bone marrow-derived hematopoietic stem cells can be collected in a less invasive method compared to the conventionally known methods. Additionally, the method for promoting migration of bone marrow-derived hematopoietic stem cells into the peripheral blood may further include a step of collecting the migrated bone marrow-derived hematopoietic stem cells, after the method for promoting migration of the hematopoietic stem cells into the peripheral blood.

### [Advantageous Effects of the Invention]

According to a method for enhancing one or more stem cell functions selected from the group consisting of mobility, an engraftment ability, a CFU-F forming ability, an anti-inflammatory immunosuppression inducing function, and an angiogenesis promoting ability of stem cells, the method can easily prepare a large amount of stem cells with excellent compatibility to human body due to a high engraftment ability, mobility, and an excellent colony-forming ability using a low-cost short-chain fatty acid derived from humans or a salt thereof, and can provide stem cells suitable for a cell therapy product by activating the stem cells into a state suitable for transplantation.

### [Brief Description of Drawings]

FIG. 1 shows the results confirming the enhancement of mobility of ST2 cells (*i.e.,* mouse bone marrow-derived MSCs) by sodium acetate treatment according to concentrations.
FIG. 2 shows the results confirming the enhancement of mobility of ST2 cells (*i.e.,* mouse bone marrow-derived MSCs) by sodium propionate or sodium butyrate treatment according to concentrations.
FIG. 3 shows the results confirming the changes in the expression level of proteins related to mobility-promoting signals in ST2 cells (*i.e.,* mouse bone marrow-derived MSCs) by sodium acetate treatment.
FIG. 4 shows the results confirming the enhancement of invasion in ST2 cells (*i.e.,* mouse bone marrow-derived MSCs) by sodium acetate treatment.
FIG. 5 shows the results confirming the enhancement of mobility of human bone marrow-derived MSCs by sodium acetate treatment.
FIG. 6 shows the results confirming the enhancement of a CFU-F forming ability of ST2 cells (*i.e.,* mouse bone marrow-derived MSCs) by sodium acetate or sodium propionate treatment.
FIG. 7 shows the results confirming the enhancement of mobility from the bone marrow to the peripheral blood in hematopoietic progenitor cells by sodium acetate treatment.
FIG. 8 shows the results confirming the enhancement of a CFU-F forming ability of human bone marrow-derived MSCs by sodium acetate treatment.
FIG. 9 shows the results confirming the increase of frequency and size of a cell population of bone marrow-derived MSCs by intake of drinking water containing sodium acetate (200 mM).
FIG. 10 shows the results confirming the increase in the expression level of proteins related to signals promoting anti-inflammatory immunosuppression and angiogenesis in ST2 cells (*i.e.,* mouse bone marrow-derived MSCs) by sodium acetate treatment.

### [Best Mode]

Hereinafter, the preferred Examples are provided for better understanding of the present invention. However, these Examples are for illustrative purposes only, and the scope of the invention is not intended to be limited by these Examples.

### <Example 1> Confirmation of promotion of mobility of mouse bone marrow-derived MSCs by treatment with short-chain fatty acid

The effect of treatment of mouse bone marrow-derived MSCs with a short-chain fatty acid on mobility promotion was confirmed by migration assay. The ST2 cell line (*i.e.,* mouse bone marrow-derived MSCs) was obtained from the Riken Cell Bank (Tsukaba, Japan). The cells were maintained in RPMI1640 medium (Invitrogen) containing 10% FBS, 1% penicillin and streptomycin (Invitrogen). The cells were cultured to a confluence of 80%, recovered with 0.25% trypsin/EDTA (Invitrogen), and subcultured in a ratio of 1 : 3 to 1 : 4. The cells which underwent 5 to 8 subcultures were used in the experiment. The cells were maintained in a 37°C incubator containing 5% CO₂.

Millicell culture plate inserts with a pore size of 8 µm (EMD Millipore) were coated with type I collagen (0.5 µg/mL). The ST2 cells (2.5 × 10⁴ cells) were suspended in normal growth medium and added into an upper chamber. After culturing the cells for 5 to 6 hours, the medium was replaced with DMEM medium containing 2% FBS and 1% penicillin and streptomycin, and the cells were cultured overnight and sodium acetate, sodium propionate, and sodium butyrate, each at a concentration of 0 mM to 10 mM, were added to each of the lower chambers (all of the short-chain fatty acids were purchased from Sigma-Aldrich).

After culturing for additional 12 hours, each insert was fixed with 4% formaldehyde in the presence of PBS at room temperature for 10 minutes. The ST2 cells were stained with a hematoxylin solution for 30 minutes, and the cells remaining on the upper chamber membrane were removed using cotton swabs. The lower chamber membrane was confirmed using the Nikon ECLIPSE TS 100 microscope. The number of cells in each cell image was counted using the Adobe Photoshop CS6.

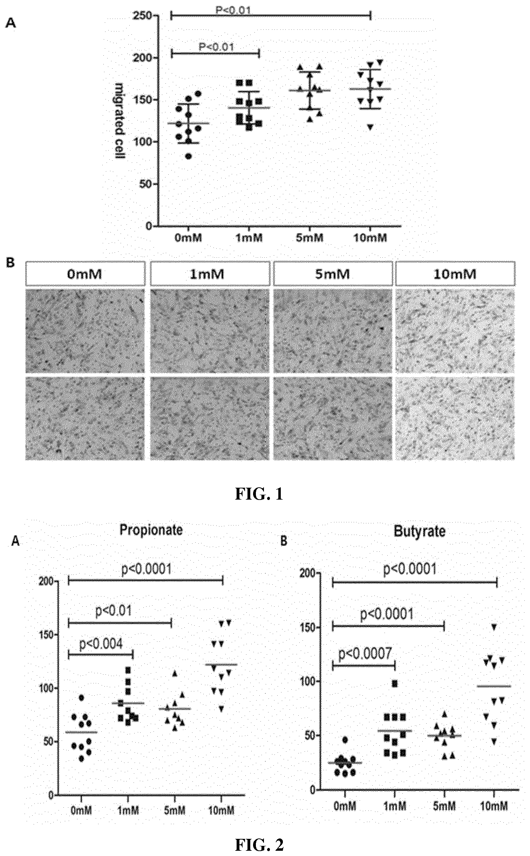

The results of migration assay performed using sodium acetate are shown in FIG. 1. FIG. 1A shows the results of counting the number of cells where migration occurred and FIG. 1B shows the microscopic image. As can be seen in FIG. 1, the mobility of ST2 cells (*i.e.,* mouse bone marrow-derived MSCs) were shown to increase in a manner dependent on the concentration of sodium acetate.

Additionally, FIG. 2 shows the results of the experiment performed using sodium propionate and sodium butyrate. As can be seen in FIG. 2, the mobility of ST2 cells were shown to increase in a manner dependent on the concentrations of sodium propionate and sodium butyrate.

### <Example 2> Confirmation of changes in signal transducer promoting mobility of mouse bone marrow-derived MSCs by treatment with short-chain fatty acid

The effect of treatment of mouse bone marrow-derived MSCs with a short-chain fatty acid on the changes in the signal transducer promoting mobility was confirmed by western blot analysis.

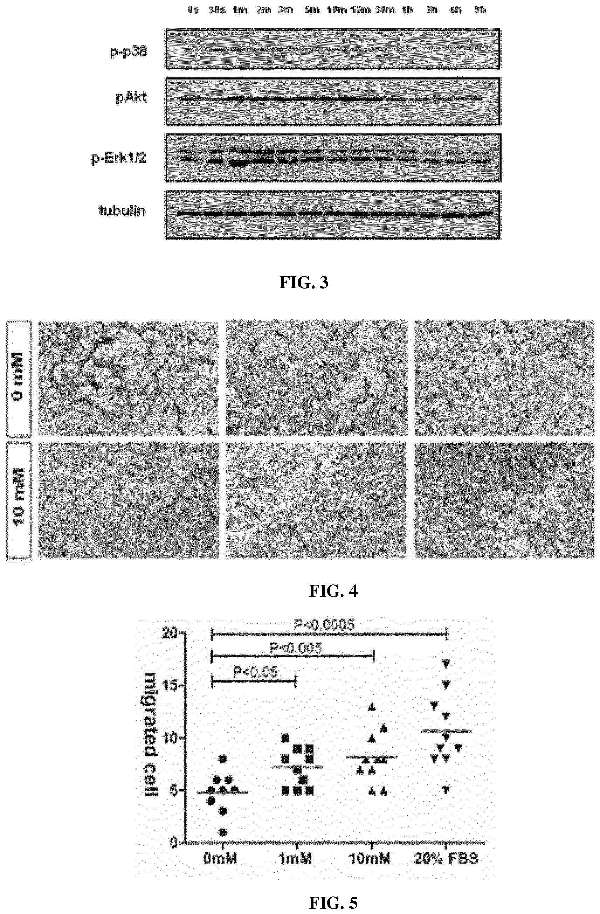

ST2 cells were added to a 6-well plate in the presence of normal growth medium. After culturing the ST2 cells, the medium was replaced with serum-free medium containing 1% penicillin and streptomycin and cultured for 16 to 18 hours. The cells were treated with 10 mM sodium acetate for 30 seconds, 1 minute, 2 minutes, 3 minutes, 5 minutes, 10 minutes, 15 minutes, 30 minutes, 1 hour, 3 hours, 6 hours, and 9 hours, respectively. Cell lysates were obtained from the above samples and protein samples were isolated by 10% SDS-PAGE. The isolated cells were transferred into a nitrocellulose membrane to block the membrane and treated with primary antibodies of phospho-p44/42 MAPK (Erk1/2) (Thr202/Tyr204) (Cell Signaling Technology), phospho-Akt (Ser473) (Cell Signaling Technology), or phospho-p38 MAPK (Thr180/Tyr182) (Cell Signaling Technology). Then, the cells were treated with HRP-conjugated secondary antibodies and the protein bands were confirmed by chemiluminescence detection. The results are shown in FIG. 3.

As can be seen in FIG. 3, it was confirmed that the signaling was activated early and transient. That is, it was confirmed that the factors associated with the promotion of cell mobility underwent changes by the treatment with a short-chain fatty acid or a salt thereof thereby promoting the mobility of the cells.

### <Example 3> Confirmation of promotion of invasion of mouse bone marrow-derived MSCs by treatment with short-chain fatty acid

The effect of treatment of mouse bone marrow-derived MSCs with a short-chain fatty acid on the promotion of invasion was confirmed by invasion assay.

Millicell culture plate inserts with a pore size of 8 µm (EMD Millipore) were coated with type I collagen (0.5 µg/mL). Then, the upper part of each insert was coated with 50 µL of growth factor reduced Matrigel (BD Bioscience). The ST2 cells (2.5 × 10⁴ cells) were suspended in their respective normal growth medium and added into an upper chamber. After culturing the cells for 5 to 6 hours, the medium was replaced with DMEM medium containing 2% FBS and 1% penicillin and streptomycin, and the cells were cultured overnight and sodium (10 mM) was added to each of the lower chambers (all of the short-chain fatty acids were purchased from Sigma-Aldrich). After culturing for additional 12 hours, each insert was fixed with 4% formaldehyde in the presence of PBS at room temperature for 10 minutes. The ST2 cells were stained with a hematoxylin solution for 30 minutes, and the cells remaining on an upper chamber membrane were removed using cotton swabs. The lower chamber membrane was confirmed using the Nikon ECLIPSE TS 100 microscope. The number of cells in each cell image was counted using the Adobe Photoshop CS6. The results are shown in FIG. 4.

As can be seen in FIG. 4, it was confirmed that the number of cells significantly increased by the sodium acetate treatment and this indicates that the invasion increased. That is, it was confirmed that the matrigel invasion ability of human bone marrow-derived MSCs was increased by 10 mM sodium acetate treatment, and this indicates that the ECM remodeling ability/migration ability of the stem cells was increased by the sodium acetate treatment.

### <Example 4> Confirmation of promotion of mobility of human bone marrow-derived MSCs by treatment with short-chain fatty acid

The effect of treatment of human bone marrow-derived MSCs with a short-chain fatty acid on the promotion of mobility was confirmed by migration assay.

The human bone marrow derived MSCs (hMSCs) were obtained from the Lonza (Basel, Switzerland) and cultured in MSCGM medium (Lonza). The cells which underwent 3 to 6 subcultures were used in the experiment. For the migration experiment of human bone marrow derived MSCs (hMSCs), DMEM medium (GE Healthcare Life Sciences) containing 10% FBS, 1% penicillin and streptomycin (Invitrogen), and 1% L-glutamine was used. The cells were maintained in a 37°C incubator containing 5% CO₂.

In the case of the human bone marrow derived MSCs (hMSCs), migration assay was performed in a similar manner as in Example 1, except that the normal medium was replaced with serum-free DMEM medium containing 1% L-glutamine, 1% penicillin, and streptomycin, after 5 to 6 hours of cultivation, and the cells remaining on the upper chamber membrane were removed, and each insert was fixed to a slide using 4',6-diamidino-2-phenylindole (DAPI) as the ProLong Gold antifade mounting solution. The results are shown in FIG. 5.

As can be seen in FIG. 5, it was confirmed that the mobility of human bone marrow derived MSCs (hMSCs) increased in a manner dependent on the concentration of sodium acetate, which is a short-chain fatty acid or a salt thereof.

### <Example 5> Confirmation of enhancement of colony-forming ability of mouse bone marrow-derived MSCs by treatment with short-chain fatty acid

The effect of treatment of mouse bone marrow-derived MSCs with a short-chain fatty acid on the enhancement of a colony-forming ability was confirmed by colony-forming unit fibroblast (CFU-F) assay.

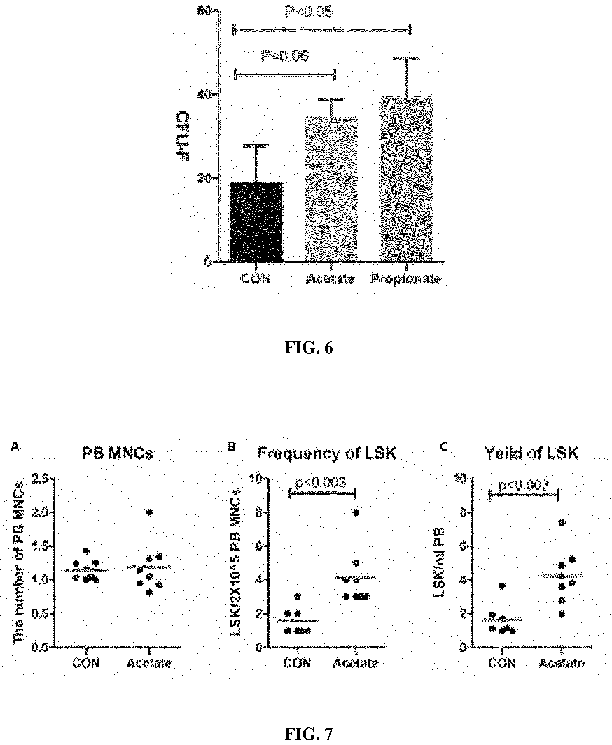

ST2 cells were treated with sodium acetate (10 mM) or sodium propionate (10 mM) and cultured in normal culture medium for 36 hours. Then, the cells were added to a plate at a concentration of 5.7 × 10⁴ cells/well and cultured under 37°C and 5% CO₂ conditions for 10 days. The plate was then stained with a 1% crystal violet staining solution (Sigma-Aldrich) and the number of colonies was counted under a stereomicroscope. In particular, cell populations with a diameter of 1 mm or greater were considered as colonies. The results are shown in FIG. 6.

As can be seen in FIG. 6, it was confirmed that the colony-forming ability of the mouse bone marrow-derived MSCs was significantly increased by the treatment of sodium acetate, which is a short-chain fatty acid or a salt thereof.

### <Example 6> Confirmation of enhancement of mobility of hematopoietic progenitor cells by treatment with short-chain fatty acid

The effect of treatment of hematopoietic progenitor cells with a short-chain fatty acid on the enhancement of mobility was confirmed by flow cytometry assay.

Populations of circulating hematopoietic progenitor cells (HPCs), lineage negative, Sca-1 positive, and c-Kit positive (Lin⁻/Sca1⁺/c-Kit⁺, LSK) cells were analyzed by a flow cytometry analyzer. Each 8-week-old C57BL/6 mouse was subcutaneously injected on its side with sodium acetate (4 nmole/kg), and after 10 minutes, the peripheral blood was collected.

Monocytes were separated by Ficoll centrifugation. Then, hematopoietic progenitor cells were examined using anti-mouse CD4-FITC (Biolegend, San Diego, CA), anti-mouse CD49b-FITC (Biolegend), anti-mouse CD11b-FITC (Biolegend), anti-mouse B220-FITC (Biolegend), anti-mouse Gr-1-FITC (Biolegend), anti-mouse CD8-FITC (Biolegend), anti-mouseLy-6A/E (Sca-1)-PE (Biolegend), and anti-mouse CD117 (c-Kit)-APC (Biolegend). The results are shown in FIG. 7.

As can be seen in FIG. 7, it was confirmed that there was no significant change in the number of monocytes of the peripheral blood (PB MNCs), however, the hematopoietic progenitor cells exhibiting the Lin⁻/Sca1⁺/c-Kit⁺ (LSK) characteristic were observed more in the peripheral blood compared to the normal control group thus confirming that the short-chain fatty acid or a salt thereof are effective for the mobility of stem cells.

### <Example 7> Confirmation of enhancement of colony-forming ability of human bone marrow-derived MSCs by treatment with short-chain fatty acid

The effect of treatment of human bone marrow-derived MSCs (hMSCs) with a short-chain fatty acid on the enhancement of a colony-forming ability was confirmed by colony-forming unit fibroblast (CFU-F) assay.

The human bone marrow-derived mesenchymal stem cells (hMSCs) were subcultured twice in normal culture medium (DMEM high glucose + 10% FBS + 1% penicillin and streptomycin) after treating with sodium acetate (10 mM). Then, the cells were added at a concentration of 5 × 10² cells/well (10 cm dish) to low-glucose DMEM (GE Healthcare Life Sciences) containing 10% FBS, 1% penicillin and streptomycin (Invitrogen), and 1% L-glutamine in a plate, and the plate was incubated under 37°C and 5% CO₂ conditions for 14 days. The plate was then fixed with 100% methanol, stained with a 1% crystal violet staining solution (Sigma-Aldrich), and the number of colonies was counted under a stereomicroscope. In particular, cell populations of human bone marrow-derived MSCs with a diameter of 3 mm or greater were considered as colonies.

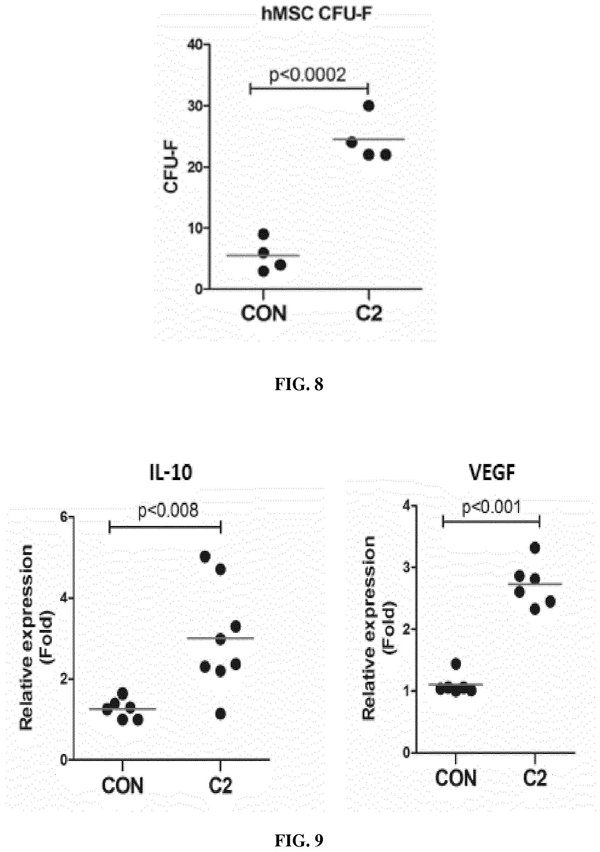

The results are shown in FIG. 8.

As can be seen in FIG. 8, it was confirmed that the human bone marrow-derived MSCs (hMSCs) showed significant enhancement of a colony-forming ability by about 5-fold by the treatment of sodium acetate, which is a short-chain fatty acid or a salt thereof.

### <Example 8> Confirmation of increase of size of cell population of mouse bone marrow-derived MSCs by intake of drinking water containing short-chain fatty acid

The effect of intake of drinking water containing a short-chain fatty acid on the increase of the size of a cell population of mouse bone marrow-derived MSCs was confirmed by colony-forming unit fibroblast (CFU-F) assay.

Each C57BL/6 mouse was fed with drinking water with or without sodium acetate (200 mM) for 7 days, and femurs were collected from each mouse. The collected femurs were crushed using a mortar, treated with 0.2% type I collagenase (Worthington), and thereby the bone marrow cells attached to the bones were collected. The collected cells were added at a concentration of 2.0 × 10⁵ cells/well (25T flask) to α-MEM (Sigma-Aldrich) containing 20% FBS, 1% penicillin and streptomycin (Invitrogen), 1% L-glutamine, and 5% pyruvic acid in a plate, and the plate was incubated under 37°C and 5% CO₂ conditions for 14 days.

The plate was then fixed with 100% methanol, stained with a 1% crystal violet staining solution (Sigma-Aldrich), and the number of colonies was counted under a stereomicroscope. In particular, cell populations with a diameter of 1 mm or greater were considered as colonies.

The results are shown in FIG. 9.

As can be seen in FIG. 9, it was confirmed that the mouse bone marrow-derived MSCs, which were collected from the mice fed with drinking water containing sodium acetate as a short-chain fatty acid or a salt thereof, showed increases in frequency and size of cell population by about 2-fold, compared to the mouse bone marrow-derived MSCs, which were collected from the mice fed with drinking water without sodium acetate.

### <Example 9> Confirmation of increase of expression level size of factors related to signals promoting anti-inflammatory immunosuppression and angiogenesis of mouse bone marrow-derived MSCs by treatment with short-chain fatty acid

The effect of treatment of mouse bone marrow-derived MSCs with a short-chain fatty acid on changes in factors related to signals promoting anti-inflammatory immunosuppression and angiogenesis of mouse bone marrow-derived MSCs was confirmed by the increase of mRNA levels.

ST2 cells (3.8 × 10⁵ cells) were added to a 6-well plate under the condition of normal growth medium and cultured overnight. The medium was replaced with low-glucose DMEM medium without FBS and the cells were cultured for 18 hours. Then, the cells were treated with 10 mM sodium acetate (C2) for 48 hours or not treated (CON).

A cell lysate was obtained from the sample, and total RNA was isolated using TRIzol. The cDNA was synthesized from the total RNA, and the expression levels of mRNAs of VEGF and IL-10 in cells were measured by real-time PCR using primers for VEGF and IL-10 and SYBR green PCR Master Mix (fluorescent material).

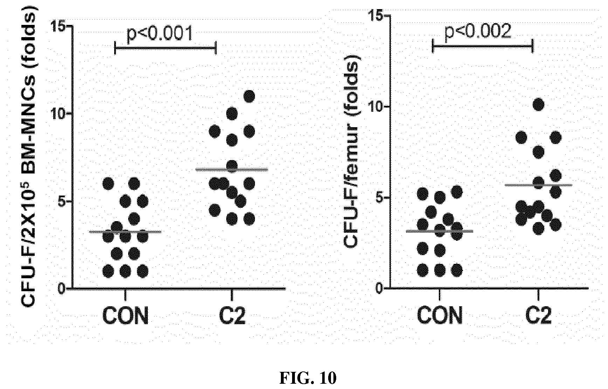

The results are shown in FIG. 10.

As can be seen in FIG. 10, it was confirmed that the expression of IL-10 exhibiting the anti-inflammatory immunosuppression function showed about a 2.4-fold increase and the expression of VEGF exhibiting the angiogenesis promotion showed about a 2.5-fold increase, compared to the control group (CON) not treated with sodium acetate, respectively. That is, it was confirmed that the treatment of stem cells with a short-chain fatty acid or a salt thereof activated the stem cells and thereby the synthesis of factors related to signals promoting anti-inflammatory immunosuppression and angiogenesis of the stem cells was enhanced.

## Claims

1. A method for enhancing one or more stem cell functions selected from the group consisting of mobility, an engraftment ability, a CFU-F forming ability, an anti-inflammatory immunosuppression inducing function, and an angiogenesis-promoting ability of stem cells, comprising treating stem cells with a short-chain fatty acid or a salt thereof.

2. The method of claim 1, wherein the short-chain fatty acid is one or more selected from acetic acid, propionic acid, butyric acid, caproic acid (C₆), caprylic acid (C₈), capric acid (C₁₀), lauric acid (C₁₂), myristic acid (C₁₄), palmitic acid (C₁₆), stearic acid (C₁₈), oleic acid (C_{18:1}) elaidic acid (C_{18:1}), linoleic acid (C_{18:2}), α-linolenic acid (C_{18:3}), γ-linolenic acid (C_{18:3}), arachidonic acid (C_{20:3}), eicosapentaenoic acid (C_{20:5}), docosahexaenoic acid (C_{22:6}), 5,6-epoxyeicosatrienoic acid, and 8,9-epoxyeicosatrienoic acid.

3. The method of claim 1, wherein the stem cells are mesenchymal stem cells or hematopoietic stem cells.

4. The method of claim 1, wherein the treatment of stem cells with the short-chain fatty acid or a salt thereof is performed *ex vivo* or *in vitro.*

5. The method of claim 1, wherein the treatment of stem cells with the short-chain fatty acid or a salt thereof is performed in serum-free DMEM medium or RPMI1640 for 3 hours to 14 days.

6. A method for enhancing one or more stem cell functions selected from the group consisting of mobility, an engraftment ability, a CFU-F formation ability, an anti-inflammatory immunosuppression inducing function, and an angiogenesis-promoting ability of stem cells, comprising a short-chain fatty acid or a salt thereof as an active ingredient.

7. The method of claim 6, wherein the short-chain fatty acid is one or more selected from acetic acid, propionic acid, butyric acid, caproic acid (C₆), caprylic acid (C₈), capric acid (C₁₀), lauric acid (C₁₂), myristic acid (C₁₄), palmitic acid (C₁₆), stearic acid (C₁₈), oleic acid (C_{18:1}) elaidic acid (C_{18:1}), linoleic acid (C_{18:2}), α-linolenic acid (C_{18:3}), γ-linolenic acid (C_{18:3}), arachidonic acid (C_{20:3}), eicosapentaenoic acid (C_{20:5}), docosahexaenoic acid (C_{22:6}), 5,6-epoxyeicosatrienoic acid, and 8,9-epoxyeicosatrienoic acid.

8. Stem cells prepared according to the method of any one of claims 1 to 5.

9. A cell therapy composition comprising the stem cell of claim 8.

10. A method for promoting migration of bone marrow-derived hematopoietic stem cells into the peripheral blood in a subject excluding humans, comprising administering a short-chain fatty acid or a salt thereof to the subject.

11. A composition for enhancing one or more stem cell functions selected from the group consisting of mobility, an engraftment ability, a CFU-F formation ability, an anti-inflammatory immunosuppression inducing function, and an angiogenesis-promoting ability of stem cells, comprising a short-chain fatty acid or a salt thereof as an active ingredient.

12. The composition of claim 11, wherein the short-chain fatty acid is one or more selected from acetic acid, propionic acid, butyric acid, caproic acid (C₆), caprylic acid (C₈), capric acid (C₁₀), lauric acid (C₁₂), myristic acid (C₁₄), palmitic acid (C₁₆), stearic acid (C₁₈), oleic acid (C_{18:1}) elaidic acid (C_{18:1}), linoleic acid (C_{18:2}), α-linolenic acid (C_{18:3}), γ-linolenic acid (C_{18:3}), arachidonic acid (C_{20:3}), eicosapentaenoic acid (C_{20:5}), docosahexaenoic acid (C_{22:6}), 5,6-epoxyeicosatrienoic acid, and 8,9-epoxyeicosatrienoic acid.

13. The composition of claim 11, wherein the stem cells are mesenchymal stem cells or hematopoietic stem cells.
